# EUROPEAN PATENT APPLICATION

(11) **EP 4 353 184 A1**
(43) Date of publication of application: **17.04.2024**
(21) Application number: 23201837.4
(22) Date of filing: 05.10.2023
(51) Int. Cl.: A61B 34/37, A61B 34/00, A61B 90/60, A61B 90/98

(54) **SURGICAL SYSTEM**

(30) Priority: 11.10.2022 JP 2022163383
(71) Applicant: Medicaroid Corporation, Kobe-shi, Hyogo 650-0047 (JP)
(72) Inventor: YOSHINO, Hiroki, Hyogo, 650-0047 (JP); FUKAI, Kentaro, Hyogo, 650-0047 (JP); KAWABATA, Hideo, Hyogo, 650-0047 (JP)
(74) Representative: Müller Hoffmann & Partner

(57) **Abstract**

One or more embodiments of the disclosure may be a surgical system that may include: a surgical robot; an operating device configured to be operated by a doctor to operate the surgical robot; and a controller. The operating device includes a component for which a setting is adjustable, and an information reader configured to acquire user information from a storage medium that stores the user information including user identification information and setting information of the component. The controller is configured control the component to adjust the setting of the component based on the setting information acquired from the storage medium by the information reader.

## Description

### BACKGROUND

The disclosure may relate to a surgical system and, more particularly, may relate to a surgical system that includes a manipulator including a component for which a setting are adjustable.

In a related art, a surgical system is known that includes an operating device including a component for which a setting is adjustable (see, for example, Patent Document 1: U.S. Patent No. 8,508,173).

U.S. Patent No. 8,508,173 discloses a surgical system that includes a surgical robot and an operating device that includes a setting adjustable component whose setting is adjustable and is configured to operate the surgical robot. The surgical system also includes a storage device that stores ergonomic settings, associated with a user's login ID, for setting adjustable components, such as a height of a display, a pedal position of a pedal system, a height of an armrest, etc. Based on the user's login ID entered via a touchpad, the ergonomic settings associated with the user's login ID are retrieved from the storage device so as to automatically adjust the height of the display, and the like.

Patent Document 1: U.S. Patent No. 8,508,173

### SUMMARY

However, in the surgical system disclosed in Patent Document 1, based on the user's login ID input through the touch pad, the ergonomic settings are read from the storage device to automatically adjust the height of the display and the like. In a medical institution that has newly introduced a surgical system, it is necessary to invite a preceptor called a proctor to perform robotic surgery (robot-assisted surgery) while receiving guidance and education on from the preceptor. In such a case, there is a problem that ergonomic setting for the preceptor cannot be stored in a storage device in advance, and it is necessary to store the ergonomic setting for the preceptor in the storage device after the preceptor arrives at the medical institution, which is troublesome.

An object of an embodiment of the disclosure is to provide a surgical system that is capable of reducing the time and effort to adjust a setting of a component for an intended user even if the setting of the component for the indented user is not stored in an operating device of the surgical system.

An aspect of the disclosure may be a surgical system that may include a surgical robot, an operating device for a doctor to operate the surgical robot, and a controller. The operating device includes a component configured to adjust a setting thereof, an information reader configured to acquire user information from a storage medium that stores user information including user identification information and component setting information. The controller is configured to, based on the setting information acquired from the storage medium by the information reader, control the component to adjust the setting of the component.

In the surgical system according to the aspect described above, the controller controls the component to adjust the setting of the component based on the setting information acquired from the storage medium by the information reader. Accordingly, even though the setting of the component of the operating device is not stored in the storage medium of the surgical system, the setting of the component can be obtained from the storage medium possessed by the doctor. As a result, it is necessary to manually input the setting of the component of the operating device. Therefore, even if the setting of the component is not stored in the operating device of the surgical system, it is possible to reduce the time and effort to adjust the setting of the component.

According to the aspect of the disclosure, it may be possible to reduce the time and effort to adjust setting of the component even when the setting of the component is not stored in the operating device of the surgical system.

### BRIEF DESCRIPTION OF DRAWINGS

FIG. 1 is a diagram illustrating a view of a configuration of a surgical system according to an embodiment;
FIG. 2 is a block diagram illustrating a view of a configuration of the surgical system according to an embodiment;
FIG. 3 illustrates a scope type display of the surgical system according to an embodiment;
FIG. 4 is a diagram illustrating a cross-sectional view taken along the line IV-IV in FIG. 1;
FIG. 5 is a diagram illustrating a cross-sectional view taken along the V-V line in FIG. 1;
FIG. 6 is a diagram illustrating a view of an example of a display screen of the surgical system according to an embodiment; and
FIG. 7 is a diagram illustrating a display example of usage history information according to an embodiment.

### DETAILED DESCRIPTION

Descriptions are provided hereinbelow for one or more embodiments based on the drawings. In the respective drawings referenced herein, the same constituents are designated by the same reference numerals and duplicate explanation concerning the same constituents is omitted. All of the drawings are provided to illustrate the respective examples only.

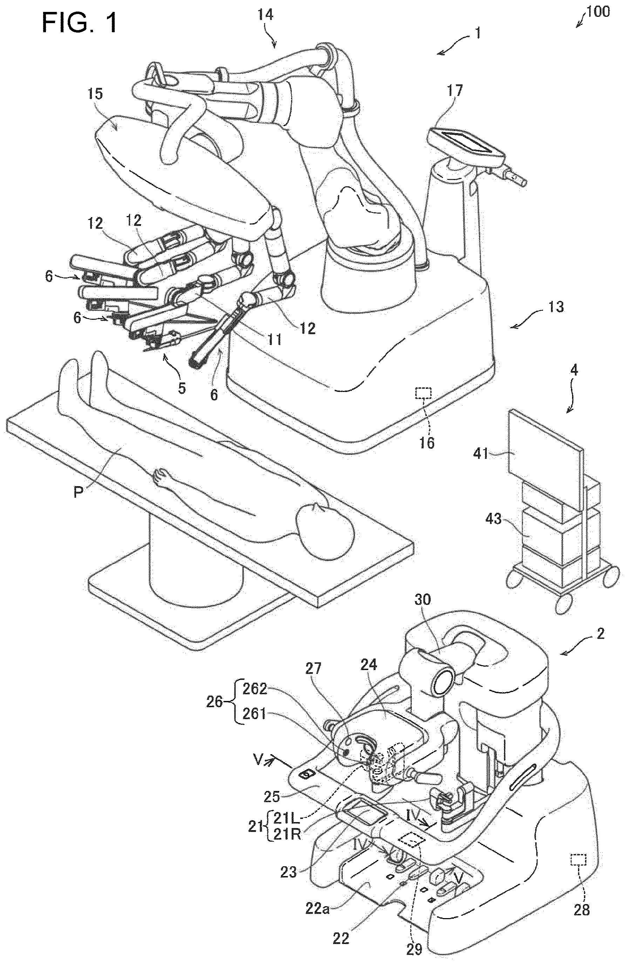

A configuration of a surgical system 100 (a surgical operation system 100) according to an embodiment is described with reference to FIGS. 1 to 7.

As illustrated in FIG. 1, the surgical system 100 includes a surgical robot 1 and an operating device 2 (control device, remote control device) to operate the surgical robot 1. The surgical robot 1 and the operating device 2 are placed or installed in a same facility. Assistants are assigned near the surgical robot 1 as surgical staff. The surgical system 100 is also equipped with a monitor cart 4 for the assistants to share information.

The surgical robot 1 includes one manipulator arm 11 that holds an endoscope 5 and a plurality (three, in this example) of manipulator arms 12 that hold surgical instruments 6. The endoscope 5 captures an image of a surgical site inside a patient P. An end effector such as forceps is attached to a distal end of each of or at least one of the surgical instruments 6. The operating device 2 is provided for operating the manipulator arms 11 and 12.

The surgical robot 1 is placed in a surgery room (or an operating room). The surgical robot 1 includes a medical cart 13 , a positioner 14 and an arm base 15. The surgical robot 1 is configured to be movable by the medical cart 13. The medical cart 13 is provided with a controller 16 (or a controller) that controls operation of the surgical robot 1. The controller 16 controls the operation of the surgical robot 1 based on commands input to the operating device 2. The controller 16 includes a processor or a circuitry such as a CPU that executes programs, and a storage such as a memory that stores the programs.

Further, the medical cart 13 is provided with an input device 17. The input device 17 is configured to accept operations to move or change postures of the manipulator arm 11, the manipulator arms 12, the positioner 14, the arm base 15, mainly in order to prepare for surgery before the surgery.

The positioner 14 is configured as a seven-axis articulated robot. The positioner 14 is disposed on the medical cart 13. The positioner 14 is configured to move the arm base 15. Specifically, the positioner 14 is configured to move the position of the arm base 15 three-dimensionally.

The arm base 15 is attached to a distal end of the positioner 14. The manipulator arm 11 and the manipulator arms 12 are attached to the arm base 15 at their base portions (proximal end portions). The arm base 15, the manipulator arm 11, and the manipulator arm 12 are used with being covered with a sterile drape.

The control device 2 is placed inside the surgery room or outside the surgery room, for example. The operating device 2 includes operating handles 21, foot pedals 22, a touch panel 23, a scope type display 24, an armrest 25, a voice communication device 26, a sensor 27, a controller 28, a reader (reading device) 29, and a support arm 30. The scope type display 24 is an example of a "display." The reader 29 is an example of an "information reader" and an "IC card reader."

The operating handles 21 are control handles provided for the operator (such as a doctor (surgeon)) to input commands. The operating handles 21 are provided for the operator (doctor) to operate the manipulator arms 11 and 12 (the endoscope 5 and the surgical instruments 6). Each of the operating handles 21 includes a plurality of links and a plurality of joints connecting the links, and each joint is provided with an encoder for detecting an amount of rotation. Each of the operating handles 21 also receives an operation amount (a movement amount) by which the operating handle 21 is operated (moved) to operate the manipulator arms 11 and 12 (the endoscope 5 and the surgical instruments 6). The operating handles 21 comprise a pair of operating handles 21 which include a left-hand operating handle 21L to be operated by the left hand of the operator (doctor) and a right-hand operating handle 21R to be operated by the right hand of the operator (doctor). The surgical instrument 6 that is held by one manipulator arm 12 is operated by the left-hand operating handle 21L, and the surgical instrument 6 that is held by another manipulator arm 12 is operated by the right-hand operating handle 21R.

The operating handles 21 are configured to set a ratio (scaling) of a movement amount of the endoscope 5 and the surgical instruments 6 with respect to the operation amount received by the operation handle 21. When the scaling ratio is set to 1/2, for example, the endoscope 5 or the surgical instruments 6 move 1/2 of the movement distance of the operating handles 21. This allows for precise fine surgery.

The plural foot pedals 22 are provided to operate the surgical instruments 6 and the endoscope 5 held by the manipulator arms 12 and 11 of the surgical robot 1. In other words, the plural foot pedals 22 are provided to perform functions related to the endoscope 5 and surgical instruments 6. The plural foot pedals 22 include a coagulation pedal, a cutting pedal, a camera pedal, and a clutch pedal. The plurality foot pedals 22 are operated by the operator's (doctor's) foot. When the camera pedal is operated, the operating handles 21 are allowed to move the endoscope held by the manipulator arm 11. At this time, the endoscope is moved by operating both the left-hand operating handle 21L and the right-hand operating handle 21R.

The plural foot pedals 22 are provided on a pedal tray 22a such that the positions of the plural foot pedals 22 can be adjusted frontward and backward. The pedal tray 22a is provided below the operating handles 21. Further, the pedal tray 22a is configured to be moved back and forth by a motor.

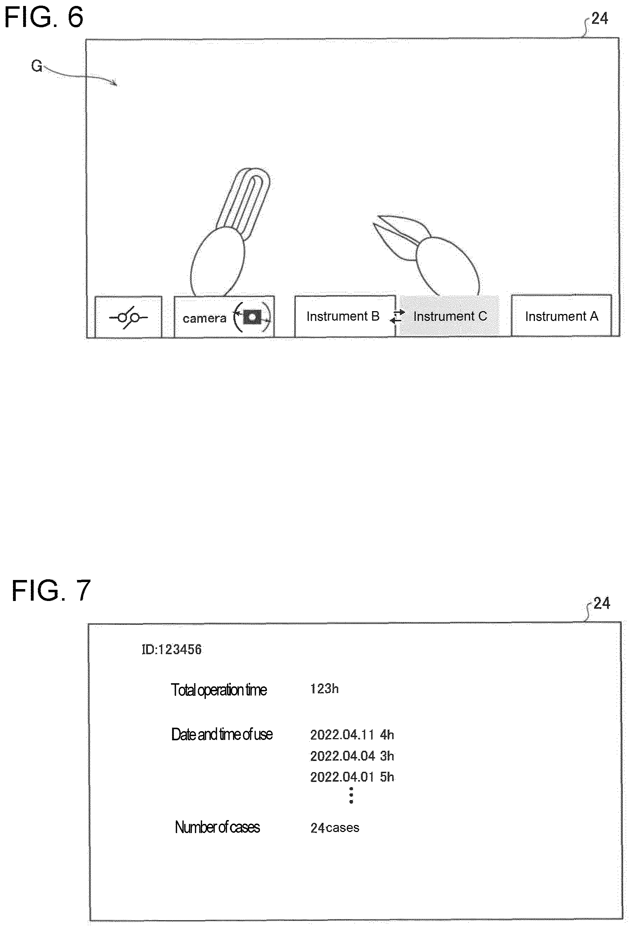

The touch panel 23 is configured to accept setting operations regarding the operating device 2. The touch panel 23 is provided to the armrest 25. The scope type display 24 is configured to display an endoscopic image (see FIG. 6) acquired by the endoscope 5. The scope type display 24 is an immersive display (display device) that allows only the operator (doctor) to see the displayed image. The operator (doctor) operates the operating handles 21 and the foot pedals 22 while viewing a surgical site (or an affected area) displayed on the scope type display 24. The support arm 30 supports the scope type display 24 so that the height of the scope type display 24 can be adjusted to the height of the face of the operator such as a doctor.

The armrest 25 is formed in a shape of a bar, and is configured so that the operator (doctor) can put his or her arm thereon when operating the operating handles 21. The voice communication device 26 is provided at a position where the operator (doctor) can perform voice communication with a voice communication device 42 of the monitor cart 4 while looking into the scope type display 24. Specifically, the voice communication device 26 is provided near the head of the operator (doctor) when the operator (doctor) looks into the scope type display 24. The voice communication device 26 is provided integrally with the scope type display 24 and thus the voice communication device 26 and the scope type display 24 can be integrally adjusted in height by the support arm 30.

The sensor 27 is configured to detect whether or not the scope type display 24 is in a state of use in which the scope type display 24 is looked into. The sensor 27 is provided at a position where the sensor 27 detects the head of the operator (doctor) when the operator positions his or her head to look into the scope type display 24. The sensor 27 is provided integrally with the scope type display 24. The controller 28 controls the operation of the operating device 2. The controller 28 includes a processor or a circuitry such as a CPU that executes programs, and a storage 28a such as a memory that stores the programs and information.

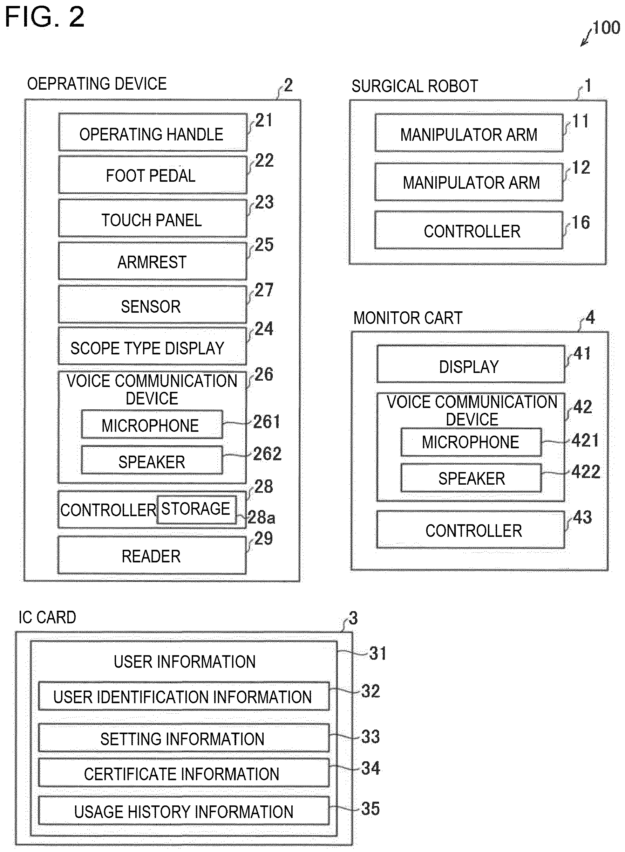

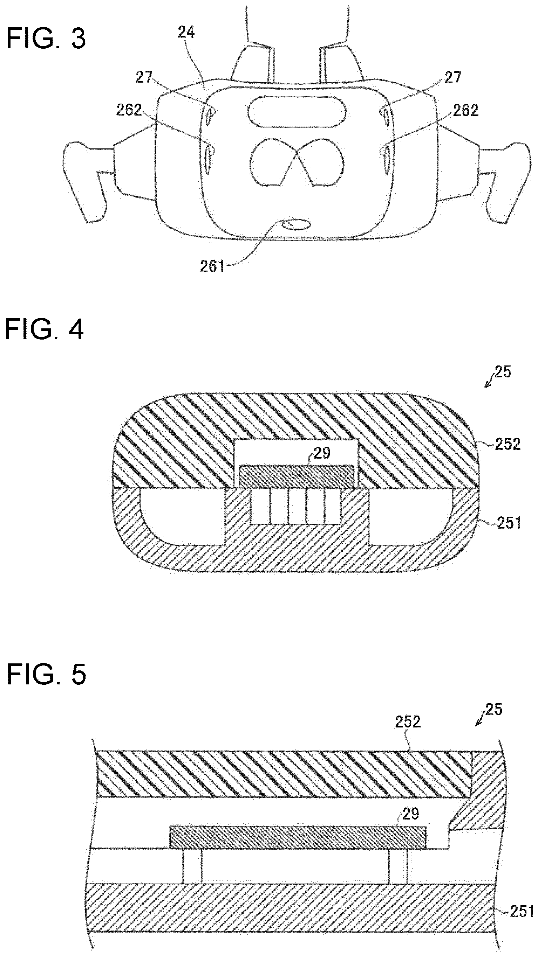

As illustrated in FIG. 2, a voice communication device 26 includes a microphone 261 and a speaker 262. The microphone 261 accepts voice input. The speaker 262 outputs sound. As illustrated in FIG. 3, the speaker 262 is provided at a position close to the operator's ear in the state where the operator (doctor) looks into the scope type display 24. The microphone 261 is provided at a position close to the operator's mouth when the operator (doctor) looks into the scope type display 24. The microphone 261 and the speaker 262 are provided integrally with the scope type display 24.

With reference to FIGS. 1 and 2, the monitor cart 4 includes a display 41 (a display device) for displaying the endoscopic image acquired by the endoscope 5 (see FIG. 1), and the voice communication device 42 for voice communication with the voice communication device 26 of the operating device 2. Also, the monitor cart 4 is positioned closer to the surgical robot 1 than the operating device 2. Therefore, an assistant placed as a surgical staff near the surgical robot 1 can check the endoscopic image through the monitor cart 4 and interact with the doctor (operator). The voice communication device 42 also includes a microphone 421 and a speaker 422. The microphone 421 accepts voice input. The speaker 422 outputs sound. The display 41 is, for example, a flat panel display or a curved panel display.

The monitor cart 4 is provided with a controller 43. An image acquired by the endoscope 5 is input to the controller 43 of the monitor cart 4. The controller 43 performs image processing. The controller 43 includes a processor or a circuitry such as a CPU that executes programs, and a storage such as a memory that stores the programs.

Note that the controller 43 displays the same image on each of the scope type display 24 and the display 41. That is, the same endoscopic image is displayed on the scope type display 24 and the display 41.

As illustrated in FIGS. 1 to 3, the operating device 2 includes the sensor 27 that detects whether or not the scope type display 24 is in the state of use, that is, whether the head of the operator is positioned to look into the scope type display 24. The controller 16 prohibits the manipulator arms 11 and 12 from being operated by the operating handles 21 when the sensor 27 does not detect the state of use, and permits the manipulator arms 11 and 12 to be operated by the operating handles 21 when the sensor 27 detects the state of use. The controller 16 is configured to, in a state where the sensor 27 does not detect the state of use, prohibit the operations of the manipulator arms 11 and 12 by not accepting input from the operating handles 21. To the contrary, the controller 16 is configured to, in a state where the sensor 27 detects the state of use state, accept predetermined inputs from the operating handles 21 by operably connecting the manipulator arms 11 and 12 to the operating handles 21.

The controller 43 is configured to generate a graphical user interface G that is superimposed on the endoscopic image. The endoscopic image on which the graphical user interface G generated by the controller 43 is superimposed is displayed on the display 41 and sent to the operating device 2 to be displayed on the scope type display 24.

Here, in an embodiment, the operating device 2 includes components whose settings can be adjusted. Also, as illustrated in FIGS. 1 and 2, the reader 29 is configured to acquire user information 31 from an integrated circuit (IC) card 3 that stores the user information 31 including user identification information 32 and component setting information 33. The controller 28 is configured to control, based on the setting information acquired from the IC card 3 through the reader 29, the components to adjust the settings of the components. Thereby, even if the settings of the components of the operating device 2 are not stored in the storage device of the surgical system 100, the settings of the components can be obtained from the IC card 3 possessed by the operator. As a result, it is not necessary for the operator to manually input the settings of the components of the operating device 2. Therefore, even if the settings of the components are not stored in the operating device 2 of the surgical system 100, it is possible to reduce or suppress the time and effort to adjust the setting of the components. Note that the IC card 3 is an example of a "storage medium."

When the surgical system 100 is activated, the controller 28 displays on the touch panel 23 a notification that prompts the user (such as a doctor) to hold the IC card 3 over the reader 29. Further, when updating the user information 31 in the IC card 3, the controller 28 displays a notification that prompts the user (such as a doctor) to hold the IC card 3 over the reader 29. For example, the user information 31 is updated when surgery or training is completed.

The component comprises one or more moveable components for each of which ergonomic setting is adjustable. Based on the setting information 33 obtained from the IC card 3 of the operator though the reader 29, the controller 28 controls the one or more movable components to move so as to adjust the ergonomic settings of the one or more movable components for the operator. Thereby, even if the ergonomic settings for the operating doctor are not stored in the operating device, it is possible to reduce the effort required to adjust the ergonomic settings for the operating doctor to the one or more movable components.

Specifically, the operating device 2 includes, as an ergonomically adjustable component, the armrest 25 of which the height is adjustable. As illustrated in FIG. 1, the reader 29 is provided on the armrest 25. As a result, the ergonomic setting of the height-adjustable armrest 25 can be easily formed according to the physique and preference of the operating doctor. Further, by providing the reader 29 on the armrest 25 which can be easily accessed by the doctor's hand, the IC card 3 can be easily brought close to the reader 29 to read the information. The operating device 2 is configured such that the height of the operating handles 21 is adjusted in conjunction with the adjustment of the height of the armrest 25.

As illustrated in FIGS. 4 and 5, the reader 29 is provided inside the armrest 25. The armrest 25 includes a lower member 251 and an upper member 252. The lower member 251 may be made of, for example, metal. The upper member 252 is made of a resin that easily transmits radio waves. The upper member 252 is formed of a member that is not too hard, since the operator's hand is placed thereon. For example, the upper member 252 is made of an elastic material.

The reader 29 is configured to read information from the IC card 3 by non-contact communication. That is, by holding the IC card 3 over the armrest 25, the operator causes the operating device 2 to acquire the user information 31. The reader 29 includes an IC card reader configured to read information stored in the IC card 3 as a storage medium. Since the IC card 3 is used as the storage medium, the doctor can easily carry the storage medium (the IC card 3).

The operating device 2 also includes the pedal tray 22a whose position is adjustable in the front-rear direction, as an ergonomically adjustable component. Thus, based on the setting information 33 acquired from the IC card 3, the operation position of the foot pedal 22 in the front-rear direction can be easily set.

The operating device 2 includes, as components whose settings are adjustable, the scope type display 24 whose brightness is adjustable, the microphone 261 whose volume is adjustable, and the speaker 262 whose volume is adjustable. Accordingly, based on the setting information 33 acquired from the IC card 3, the brightness of the scope type display 24, and the volume of the microphone 261 and the volume of the speaker 262 can be easily set. Further, the scope type display 24 is configured to adjust the contrast thereof, and the contrast of the scope type display 24 can be easily set based on the setting information 33 acquired from the IC card 3. The microphone 261 and the speaker 262 are configured to be set to be ON or OFF, and can be easily set to be turned ON or OFF based on the setting information 33 acquired from the IC card 3.

The operating device 2 also includes the operating handles 21 as setting adjustable components. The operating handle 21 is adjustable for scaling, which is the ratio of the movement amount of the surgical robot 1 to the operation amount of the operating handle 21. Based on the setting information 33 acquired from the IC card 3, the scaling of the operating handles 21 can be easily set.

As illustrated in FIG. 1, the armrest 25 is provided with the touch panel 23. The touch panel 23 is configured to receive input of settings for components. Accordingly, by operating the touch panel 23 of the armrest 25, the settings of the components can be easily done.

The reader 29 has a function of writing information to the IC card 3. That is, the reader 29 also functions as a writer (a reader/writer) The controller 28 controls the reader 29 to write, to the IC card 3, the settings for the components received through the touch panel 23. Accordingly, the setting conditions of the components input through the touch panel 23 can be stored in the IC card 3 and carried. When another operating device 2 is to be used, the same settings are easily set to the another operating device 2.

The scope type display 24 is configured such that one of a plurality of screen layouts of the scope type display 24 is selectable. The setting items of the scope type display 24 as a setting adjustable component include selection of a screen layout of the scope type display 24. This makes it possible to easily set the screen layout of the scope type display 24 based on the setting information 33 acquired from the IC card 3. Further, as illustrated in FIG. 6, the scope type display 24 is configured to display the graphical user interface G with being superimposed on the endoscope image captured by the endoscope 5. The scope type display 24 is configured such that the setting of the graphical user interface G to be superimposed is adjustable.

For example, the design of the graphical user interface G can be set. The color of the graphical user interface G can be set. The sizes of the icons in the graphical user interface G can be set. ON and OFF of the display of supplementary information, such as the time and etc., to be displayed on the graphical user interface G can be switched. ON and OFF of the display of the position of the out-of-field forceps to be displayed on the graphical user interface G can be switched. ON and OFF of the display of the level which is to be displayed when the endoscope 5 is moved can be switched. ON and OFF of the display, on the scope type display 24, of the number of times the forceps have been used can be switched.

Further, in an embodiment, the controller 28 (16) determines whether or not certificate information 34 for a robot-assisted endoscopic surgery given to the doctor is stored in the IC card 3 and permits the usage of the operating device 2 and the surgical robot 1 when it is determined that the certificate information 34 is stored in the IC card 3. As a result, it is possible to prevent use of the surgical robot 1 when it is inappropriate for the doctor to perform a robot-assisted endoscopic surgery by using the surgical system 100.

The certificate information 34 is information of a certificate issued to a doctor who has attended a training program provided by the manufacturer of the surgical robot 1, and may include, for example, a certificate number.

The certificate information 34 includes information on a plurality of certificates issued to a doctor who has attended a plurality of training programs respectively set for a plurality of surgical fields. For example, a certificate is issued to a doctor who has completed a training program for operating the surgical robot 1 for each surgical site and each surgical procedure. The controller 28 (16) is configured to permit usage of the operating device 2 and the surgical robot 1 in the fields of surgery for which the certificates contained in the certificate information 34 have been issued. Accordingly, it is possible to prevent the operating device 2 and the surgical robot 1 from being used in a surgical field in which a training program using the surgical system 100 has not been taken by the doctor.

Further, the controller 28 (16) is configured to restrict a part of the functions of the operating device 2 and the surgical robot 1 when the certificate information 34 is not stored in the IC card 3. As a result, unlike the case where all the functions of the operating device 2 and the surgical robot 1 are restricted, a person other than the doctor who do not have the certificate(s) can perform operations other than surgery such as preparing the surgical robot 1. In addition, doctors or the like who do not have the certificate are restricted from operating the operating device 2 and the surgical robot 1 for surgery or the like. For example, when the certificate information 34 is not stored, selection of uncertified types of surgery is prohibited, the types of surgical instruments 6 that can be used are limited, and/or the movement ranges of the manipulator arms 12 are limited.

As illustrated in FIG. 7, when the usage history information 35, of the doctor, of the surgical robot 1 is stored in the IC card 3, the controller 28 displays the usage history information on the scope type display 24. This allows the doctor to easily check the usage history of the surgical robot 1.

Specifically, the usage history information includes information on at least one of the total operating time of the surgical robot 1, the dates and times of use of the surgical robot 1, and the number of cases in which surgery has been performed using the surgical robot 1. This allows the doctor to easily check the total operating time of the surgical robot 1, the dates and times of use of the surgical robot 1, or the number of cases in which the surgical robot 1 has been used.

Further, the controller 28 controls the reader 29 to write information to the IC card 3 so as to update the usage history information stored in the IC card 3. Accordingly, by writing the information in the IC card 3 after the doctor has performed surgery using the surgical system 100, the usage history information can be easily updated.

### (Effects of embodiments)

One or more embodiments described above may achieve effects as described below.

In an embodiment as described above, the controller 28 is configured to control, based on the setting information acquired from the IC card 3 by the reader 29, the component(s) so as to adjust the setting(s) of the component(s). Thereby, even if the setting(s) of the component(s) of the operating device 2 is not stored in the storage device of the surgical system 100, the setting(s) of the component(s) can be obtained from the IC card 3 carried by the doctor. As a result, it is not necessary for the operator to manually input the setting(s) of the component(s) of the operating device 2. Therefore, even if the setting(s) of the component(s) is not stored in the operating device 2 of the surgical system 100, it is possible to reduce or suppress the time and effort to adjust the setting(s) of the component(s).

### (Modifications)

Note that one or more embodiments disclosed herein should be considered as exemplary in all respects and do not limit the invention. The scope of the invention is indicated by claims, not by explanation of one or more embodiments described above, and includes equivalents to the claims and all alterations (modification) within the same.

For example, in one or more embodiments described above, the case has been described in which the scope type display is provided to the operating device. However, the disclosure is not limited thereto. For example, the operating device may be provided with a display other than the scope type display, such as a curved panel display, a flat panel display, or the like.

Further, in one or more embodiments described above, the case has been described in which the voice communication is performed through the voice communication device provided in the monitor cart. However, the disclosure is not limited thereto. In the disclosure, the surgical robot may be provided with a voice communication device for voice communication.

Further, in one or more embodiments described above, the case has been described in which the IC card is used as a storage medium and the reader for reading the information in the IC card is used as an information reader. However, the disclosure is not limited thereto. In the disclosure, as a storage medium, any storage medium other than the IC card may be used. For example, as a storage medium, a mobile terminal such as a smartphone capable of contactless communication may be used. Further, as a storage medium, a storage medium such as a USB memory, an SD card, or the like, which is to be connected to communicate information, may be used. Furthermore, as a storage medium, a magnetically readable card type storage medium may be used.

Further, in one or more embodiments described above the case has been described in which the reader serving as an information reader is provided to the armrest. However, the disclosure is not limited thereto. In the disclosure, the information reader may be provided to a prat other than the armrest. For example, the information reader may be provided on a display or on a support arm that supports the display.

Further, in one or more embodiments described above, the case has been described in which examples of a movable component are the armrest and the pedal tray. However, the disclosure is not limited thereto. In the disclosure, the position of the display may be movable by a driver or a driving device, and thus the display may be a movable component. Further, the position of the operating handles may be adjustable independently of the height adjustment of the armrest, and thus the operating handles may be movable components. Alternatively, a chair with adjustable height and angle may be provided as a movable component.

Further, in one or more embodiments described above, the case has been described in which the surgical robot and the operating device are installed in the same facility. However, the disclosure is not limited thereto. In the disclosure, a surgical robot and a first operating device may be placed at a first facility and a second operating device may be placed at a second facility, so as to provide a remote surgery support from the second facility to operate the surgical robot placed at the first facility with the second operating device placed at the second facility via an external network.

### (Aspects)

It may be appreciated by those skilled in the art that one or more embodiments described above may be specific examples of the following aspects.
(Item 1) A surgical system including:
   a surgical robot;
   an operating device configured to be operated by a doctor to operate the surgical robot; and
   a controller, wherein
   the operating device includes a component for which a setting is adjustable, and an information reader configured to acquire user information from a storage medium that stores user information including user identification information and setting information of the component, and
   the controller is configured control the component to adjust the setting of the component based on the setting information acquired from the storage medium by the information reader.
(Item 2) The surgical system according to Item 1, wherein
   the component comprises one or more moveable components for each of which ergonomic setting is adjustable;
   the controller is configured to control to move the one or more movable components so as to adjust the ergonomic setting of the one or more movable components based on the setting information obtained from the storage medium by the information reader.
(Item 3) The surgical system according to Item 2, wherein
   the one or more movable components include a height-adjustable armrest, and
   the information reader is provided to the armrest.
(Item 4) The surgical system according to Item 3, wherein
   the armrest is provided with a touch panel, and
   the touch panel is configured to accept input of a setting for the component.
(Item 5) The surgical system according to Item 4, wherein
   the information reader is configured to include a function of writing information to the storage medium, and
   the controller is configured to control the information reader to write the setting for the component received by the touch panel to the storage medium.
(Item 6) The surgical system according to any one of Items 2 to 5, wherein
   the one or more movable components includes a pedal tray a position of which is adjustable frontward and rearward, and
   the pedal tray is provided with a plurality of foot pedals for manipulating a surgical instrument held by a manipulator of the surgical robot.
(Item 7) The surgical system according to any one of Items 1 to 6, wherein
   the component comprises a display with adjustable brightness, a microphone with adjustable volume, and a speaker with adjustable volume.
(Item 8) The surgical system according to any one of Items 1 to 7, wherein
   the component comprises an operating handle configured to be operated to move the surgical robot, and the setting includes scaling which is a ratio of an amount of movement of the surgical robot to an amount of movement of the operating handle.
(Item 9) The surgical system according to Item 7, wherein
   the display is configured such that, from a plurality of screen layouts, one of the plurality of screen layouts is selectable, and
   a setting item for the display include selection of the screen layout.
(Item 10) The surgical system according to any one of Items 1 to 9, wherein
   the controller is configured to determine whether or not information of a certificate of a robot-assisted endoscopic surgery given to the doctor is stored in the storage medium, and to permit to use the operating device and the surgical robot when it is determined that the information of the certificate is stored in the medium.
(Item 11) The surgical instrument according to Item 10, wherein
   the certificate comprises a plurality of certificates for a plurality of surgical fields respectively, and
   the controller is configured, for each of surgical fields for which the information of the certificate is stored in the storage medium, to permit use of the operating device and the surgical robot.
(Item 12) The surgical system according to Item 10 or 11, wherein
   the controller is configured, when it is determined that the information of the certificate is not stored in the storage medium, to restrict a part of the functions of the operating device and the surgical robot.
(Item 13) The surgical system according to any one of Items 10 to 12, wherein
   the certification is issued by attending a training program provided by a manufacturer of the surgical robot.
(Item 14) The surgical system according to any one of Items 1 to 13, wherein
   the operating device includes a display, and
   the controller is configured, when usage history information of the surgical robot used by the doctor is stored in the storage medium, to display the usage history information on the display.
(Item 15) The surgical system according to Item 14, wherein
   the usage history information includes information on at least one of a total operating time of the surgical robot, dates and times of use of the surgical robot, and a number of cases in which surgery has been performed using the surgical robot.
(Item 16) The surgical system according to Item 14 or 15, wherein
   the information reader is configured to include a function of writing information to the storage medium, and
   the controller is configured to control the information reader to write information to the storage medium so as to update the usage history information stored in the storage medium.
(Item 17) The surgical system according to any one of Items 1 to 16, wherein
   the information reader comprises an IC card reader configured to read information stored in an IC card as the storage medium.

The invention includes other embodiments or modifications in addition to one or more embodiments described above without departing from the spirit of the invention. One or more embodiments described herein are to be considered in all respects as illustrative, and not restrictive. The scope of the invention is indicated by the appended claims rather than by the foregoing description. Hence, all configurations including the meaning and range within equivalent arrangements of the claims are intended to be embraced in the invention.

## Claims

1. A surgical system comprising:
a surgical robot;
an operating device configured to be operated by a doctor to operate the surgical robot; and
a controller, wherein
the operating device includes a component for which a setting is adjustable, and an information reader configured to acquire user information from a storage medium that stores the user information including user identification information and setting information of the component, and
the controller is configured control the component to adjust the setting of the component based on the setting information acquired from the storage medium by the information reader.

2. The surgical system according to claim 1, wherein
the component comprises one or more moveable components for each of which ergonomic setting is adjustable, and
the controller is configured to control movement of the one or more movable components so as to adjust the ergonomic setting of the one or more movable components based on the setting information obtained from the storage medium by the information reader.

3. The surgical system according to claim 2, wherein
the one or more movable components comprise a height-adjustable armrest, and
the information reader is provided at the armrest.

4. The surgical system according to claim 3, wherein
the armrest is provided with a touch panel, and
the touch panel is configured to receive input of *a setting* for the one or more movable components.

5. The surgical system according to claim 4, wherein
the information reader is configured to include a function of writing information to the storage medium, and
the controller is configured to control the information reader to write the setting of the component received by the touch panel to the storage medium.

6. The surgical system according to any one of claims 2 to 5, wherein
the one or more movable components comprises a pedal tray a position of which is adjustable frontward and rearward, and
the pedal tray is provided with a plurality of foot pedals for manipulating a surgical instrument held by a manipulator of the surgical robot.

7. The surgical system according to any one of claims 1 to 6, wherein
the component comprises a plurality of components comprising a display with adjustable brightness, a microphone with adjustable volume, and a speaker with adjustable volume.

8. The surgical system according to any one of claims 1 to 7, wherein
the component comprises an operating handle configured to be operated to move the surgical robot, and the setting includes scaling, which is a ratio of a movement amount of the surgical robot to a movement amount of the operating handle.

9. The surgical system according to claim 7, wherein
the display is configured such that, among a plurality of screen layouts of the display, one of the plurality of screen layouts is selectable, and
a setting item of the display includes selection of a screen layout of the display.

10. The surgical system according to any one of claims 1 to 9, wherein
the controller is configured to determine whether or not information of a certificate of a robot-assisted endoscopic surgery given to the doctor is stored in the storage medium, and to permit to use the operating device and the surgical robot when it is determined that the information of the certificate is stored in the medium.

11. The surgical system according to claim 10, wherein
the certificate is issued for each of a plurality of surgical fields, and
the controller is configured to permit use of the operating device and the surgical robot for a surgical field for which the information of the certificate is stored in the storage medium.

12. The surgical system according to claim 10, wherein
the controller is configured, when it is determined that the information of the certificate is not stored in the storage medium, to restrict a part of the functions of the operating device and the surgical robot.

13. The surgical system according to any one of claims 1 to 12, wherein
the operating device includes a display, and
the controller is configured, when usage history information of the surgical robot used by the doctor is stored in the storage medium, to display the usage history information on the display.

14. The surgical system according to claim 13, wherein
the usage history information includes information on at least one of a total operating time of the surgical robot, dates and times of use of the surgical robot, and a number of cases in which surgery has been performed using the surgical robot.

15. The surgical system according to claim 13, wherein
the information reader is configured to include a function of writing information to the storage medium, and
the controller is configured to control the information reader to write information to the storage medium so as to update the usage history information stored in the storage medium.
